Europäisches Patentamt

European Patent Office

Office européen des brevets

(11) Publication number: **0 516 901 A1**

## EUROPEAN PATENT APPLICATION

(21) Application number: **91305125.6**

(22) Date of filing: **06.06.91**

(51) Int. Cl.5: **A01N 1/02, A61K 37/02**

(43) Date of publication of application:
**09.12.92 Bulletin 92/50**

(84) Designated Contracting States:
**AT BE CH DE DK ES FR GB GR IT LI LU NL SE**

(71) Applicant: **Lindstrom, Richard L.**
**20050 Lakeview Avenue**
**Excelsior, Minnesota 55331(US)**
Applicant: **SKELNIK, Debra L.**
**PO Box 758, Route 1**
**Cambridge, Minnesota 55008(US)**

(72) Inventor: **Lindstrom, Richard L.**
**20050 Lakeview Avenue**
**Excelsior, Minnesota 55331(US)**
Inventor: **SKELNIK, Debra L.**
**PO Box 758, Route 1**
**Cambridge, Minnesota 55008(US)**

(74) Representative: **Parr, Ronald Edward R.E. Parr**
**& Co.**
**Colman House Station Road**
**Knowle Solihull West Midlands B93 0HL(GB)**

(54) Method and apparatus of a serumfree medical solution.

(57) A serumfree medical solution for applications in Ophthalmology, that contains one or more growth factors which maintains and enhances the preservation of eye tissues, including human corneal tissues at low temperatures (2°C to 15°C). This solution is composed of a an aqueous nutrient and electrolyte solution, supplemented with a glycosaminoglycan(s), a deturgescent agent(s), a buffer system(s), an energy source(s), an antioxidant(s), membrane stabilizing components, and containing one or more growth factors.

EP 0 516 901 A1

Rank Xerox (UK) Business Services

Background of the Invention

**1. Field of the Invention:**

The present invention relates to the preservation of eye tissue in a nutritive. aqueous medical solution, and more particularly, relates to the preservation and enhancement of human corneal tissue specified as the time between removal from the donor and transplantation.

**2. Description of the Prior Art:**

Keratoplasty, or transplantation of the cornea has been effective in providing visual rehabilitation to many who suffer from cornea disorders. This procedure has gained widespread acceptance but has been severely hampered by the universally inconsistent availability of donor tissue. This problem made the development of a storage solution imperative. The development of MK™-preservation medium, and subsequent chondroitin sulfate-containing media, has positively impacted the availability of quality donor tissue. Much research in this area has been undertaken with a view towards prolonging donor storage time and yet maintaining a viable endothelium, which is crucial to successful transplantation. Storage of the cornea for up to 14 days has been reported, although the current technology does not permit adequate tissue preservation beyond a few days. Storage longer than 96 hours is attended by epithelial decomposition, and loss of cornea clarity as demonstrated by increased swelling of the cornea stroma. This stromal edema is attributed to the decreased maintenance of the barrier pump function of the corneal endothelium, a specific cell layer lining the corneal stroma.

The functional status of the endothelium and sustained corneal deturgescence after corneal preservation are of great clinical importance, and contribute primarily to the success of the surgical outcome. The ability of the cornea to maintain a relatively dehydrated state is essential to the maintenance of corneal transparency. Corneal deturgescence is an energy-dependent phenomenon performed primarily by the endothelial cells. In order for the cornea to remain viable, various enzymatic reactions must occur to carry out energy-dependent functions, maintained by high levels of ATP.

The lower temperature of the 4°C corneal storage method reduces the metabolic rate of the cornea, but the storage medium must still be able to support the basal requirements of the cornea. Corneal storage media are a complex mixture of balanced salts, amino acids, energy sources, antioxidants, buffering agents, cell membrane stabilizers, glycosaminoglycans, deturgescents and antibiotics. Temperature reduction changes the membrane lipids, proteins and water structures, each of which could alter the active transport mechanism by hindering the ease of passive diffusion, carrier-substrate interaction and energy-coupling relationships. Thus disturbances of membrane function as well as morphological and biochemical alterations may be of greater consequence as the direct result of the lower metabolic rate. Parameters for extended 4°C storage should be defined as to the reversibility of cell damage incurred during storage.

Adult corneal endothelium have a limited regenerative capacity. Mitotic figures have been rarely observed in vivo. Human corneal endothelium in vivo normally responds to trauma by sliding into the wounded area by cell migration. In vivo endothelial cell mitosis has been demonstrated in rabbits, cats and primates In tissue culture, mitosis has been observed in rabbits and human cornea endothelium Autoradiographic thymidine uptake studies after cryowounding or mechanical wounding of corneas in vitro has demonstrated existence of mitotic figures in the endothelial monolayer. Surgical trauma and disease can accelerate the loss of endothelial cells and further compromise the cornea. Thus, the long term preservation and enhancement of the corneal endothelium is a very important aspect of eye bank storage of eye tissue.

An overview of the issues surrounding the storage and handling of corneal tissue is found in Cornea Surgery, Chapters 1-4 , pages 1-128 edited by Federick S. Brightbill, M.D., published by C.V. Mosby Company, St. Louis, MO,1986. A variety of storage media and techniques have been proposed, and current research continues to be directed towards maintaining and actually enhancing the quality of donor tissues, and increasing tie duration of storage corneal tissues, as defined as the time between excision from a donor and transplantation.

Accordingly, the present invention is directed toward materials and methods for enhancing ocular tissues, especially cornea tissues, during storage prior to transplantation. One aspect of the invention provides for the enhancement of cornea tissue by stimulating endothelial cell mitosis following storage. Another aspect of the invention provides for increasing the length of time that eye tissues, especially corneal tissues can maintain the attributes of fresh tissue.

**Brief Description of the Figures**

**Figure 1:** Shows the dose response curve of bovine insulin to stimulate $^3$H-thymidine incorporation into the DNA of human corneal endothelial cells in vitro.

**Figure 2:** Shows the dose response curve of bovine insulin and human recombinant insulin to stimulate $^3$H-thymidine incorporation into the DNA of human corneal endothelial cells in vitro.

**Figure 3:** Shows the dose response curve of mEGF to stimulate $^3$H-thymidine incorporation into the DNA of human corneal endothelial cells in vitro.

**Figure 4:** Shows the dose response curve of mEGF, recombinant hEGF and insulin to stimulate $^3$H-thymidine incorporation into the DNA of human corneal endothelial cells in vitro.

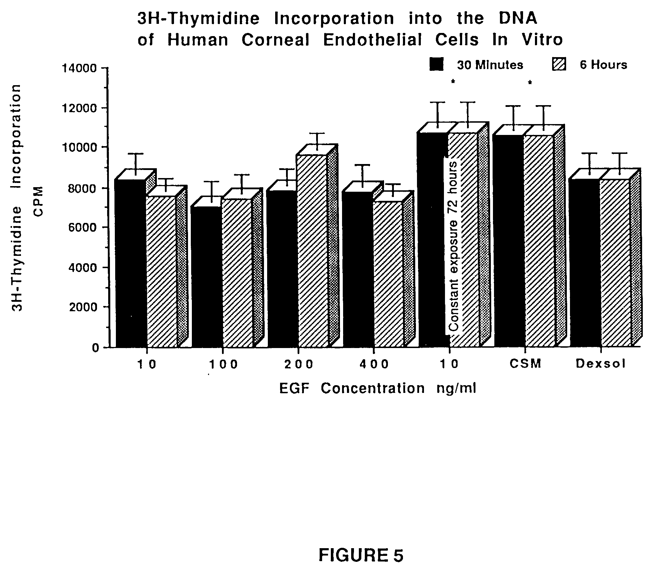

**Figure 5:** Shows the dose response curve of hEGF to stimulate $^3$H-thymidine incorporation into the DNA of human cornea] endothelial cells in vitro with respect to time.

**Figure 6:** Shows the dose response curve of insulin-like growth factor I (IGF-I) and insulin-like growth factor II (IGF-II) to stimulate $^3$H-thymidine incorporation into the DNA of human corneal endothelial cells in vitro.

**Figure 7:** Shows the post-operative percent change in endothelial cell density of human corneas transplanted into patients after storage in corneal preservation solution containing 10 ng/ml mEGF and 5 μg/ml insulin.

**Figure 8:** Shows the postoperative corneal thickness of human corneas transplanted into patients after storage in corneal preservation solution containing 10 ng/ml mEGF and 5 μg/ml insulin.

**Figure 9:** Shows the intraocular pressure of patients transplanted with human corneas stored in corneal preservation solution containing 10 ng/ml mEGF and 5 μg/ml insulin.

**Figures 10A, 10B, 10C:** Shows a detailed comparison of the media components of TC199, CSM™ Dexsol™, and K-Sol™ and M-K™.

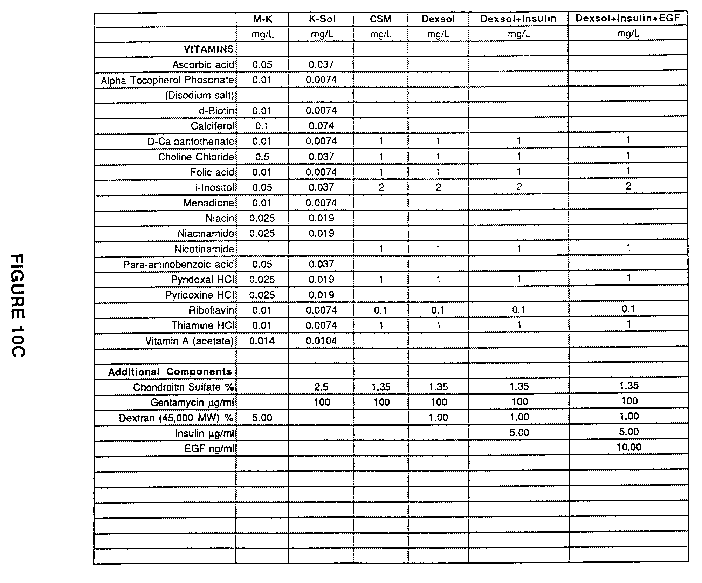

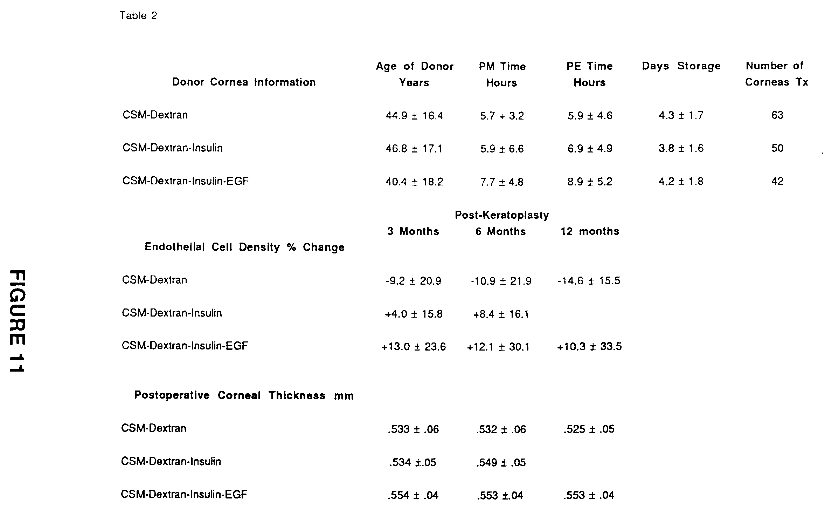

**Figure 11:** Shows a detailed comparison of postoperative patient data from corneas stored in Dexsol™, Dexsol™ supplemented with 5 μg/ml of insulin and Dexsol™ supplemented with 10 ng/ml EGF and 5 μl/ml of insulin.

## Summary of the Invention

Intermediate-term corneal storage at 4°C should provide tissue preservation which is capable of sustaining the functional status of the endothelium. Experimental work has demonstrated that both human and animal eye tissues, especially corneas, are protected from deterioration and are actually enhanced during eyebank low temperature storage in a serum-free, growth factor-containing preservation solution. The undesirable attributes of storage in serum-containing solutions are avoided, and the potential of the corneal endothelial cells to mitose following transplantation is greatly enhanced.

Growth factors (GF), which may by employed in the compositions and methods of this invention include polypeptides or other substances possessing the ability to enhance corneal wound healing, reduce or eliminate the normal progressive loss of endothelial cells, and to stimulate the mitotic potential of these ocular tissues when applied thereto. A growth factor can be tissue specific and selective as to the extent of stimulation it can cause in responsive cell types. For example, epidermal growth factor (EGF), fibroblastic growth factor (FGF), platelet-derived growth factor (PDGF), transforming growth factor alpha (TGF-α), transforming growth factor beta (TGF-β), insulin-like growth factor I, (IGF-I), insulin-like growth factor II, (IGF-II) and insulin are all agents which possess the ability to stimulate the division or maturation of certain cell types. Each of these proteins enhance, to varying degrees, activities in cell types other than those from which they were derived.

With the reduced metabolic activity at 4°C, human corneal endothelial cells do not undergo mitosis during low temperature storage in corneal preservation medium. In accordance with the present invention, it has now been discovered that corneal preservation solution that contains one or more growth factors, such as epidermal growth factor (EGF), insulin-like growth factor I (IGF-I), fibroblastic growth factor (FGF), transforming growth factor alpha (TGF-α), transforming growth factor beta (TGF-β ), platelet derived growth factor (PDGF) and insulin and the like provide excellent preservation of cornea tissues at the preferred eye bank low storage temperatures of 2°C to 15°C. Endothelial cell mitosis during such low temperature storage does not occur. When corneas are stored at 4°C in growth factor-supplemented preservation solution, and warmed to body temperature of 34°C to 37°C, however, DNA synthesis (indicative of mitosis) is stimulated and the ocular tissue is thereby enhanced. The growth factors may bind to both the corneal epithelium, endothelium or other tissues after coming into contact with the growth-factor containing solution. In addition, the cornea acts like a sponge, slowly imbibing growth factor-containing solution into the corneal stroma. After transplantation the cornea slowly releases the preloaded solution and growth factor from the stroma and surrounding tissues into the anterior chamber of the patient's eye. The growth factor is present in the solution in an amount sufficient to enhance (i.e. to stimulate mitosis following low temperature storage, especially postoperatively) human ocular cells. Although the effects of the serumfree medical solution containing a growth factor(s) is not limited to human ocular cells.

Many cells are terminally differentiated and are unable to undergo mitosis. However, there are many differentiated cells that retain the ability to return to the cell cycle if provided with an appropriate mitogenic signal, usually in the form of specific growth factor. Cell growth is carefully regulated to ensure that sufficient cells are produced during development and that cells re-enter the cell cycle at appropriate times commensurate with requirements for new cells and for body repair and other functions. The wounded state of the endothelium, as a result of trephination and trauma associated with the transplant procedure, primes the endothelium and enables it to respond to a growth factor stimulus.

Growth factors do require time to act. Although they can switch on the transcription of certain genes within minutes, such short stimulations are usually not adequate to induce DNA synthesis. The growth factor must act for several hours, which presumably means that it must be capable of activating the signal pathway for a protracted period. This need for a prolonged stimulus could possibly be the basis for the synergistic interactions that exist between various growth factors. When growth factors combine with, cell surface receptors, they entrain a sequence of events that gradually commits the cell to enter DNA synthesis. Therefore, the slow release of growth factor from the cornea over a period of days may provide the continuous stimulation needed to commit these endothelial cells to undergo limited cell division.

The novel growth factor-containing solutions are serumfree. While serum-supplemented solution can stimulate limited mitosis in human corneal endothelial cells in tissue culture, the presence of serum in products for use with tissues for human transplantation presents many disadvantages. Serum can be an agent for the transmission of diseases, such as viral diseases. Non-human-derived sera contains many substances capable of eliciting an immune response, and all sera contain some substances such as endotoxins, and growth factors that actually retard cell mitosis. These disadvantages are avoided by the present, serum-free solution.

The application of recombinant DNA technology to the production of growth factor proteins has now made it possible to formulate chemically defined growth factor-supplemented corneal preservation solution for comparative evaluation of tissue enhancement and storability. While recombinant growth factors are preferred, growth factors derived from natural biological sources are useful in the present invention.

Epidermal growth factor (EGF), acidic and basic fibroblastic growth factor (FGF), transforming growth factor alpha (TGF-$\alpha$) and transforming growth factor beta (TGF-$\beta$), platelet-derived growth factor (PDGF), insulin, and insulin-like growth factor I (IGF-I) are known proteins whose properties and biological activates have been well characterized. These peptide growth factors are described, for example in the recent review articles by Krisis et al., Biotechnology, February, 1985, pp. 135-140 and in Hormonal Proteins and Peptides, Ed. by Choh Hao Li, Vol. 12, "Growth Factors" Academic Press (1984).

EGF is a low molecular weight protein (6040 mw) previously isolated from mouse salivary glands according to the method of Savage and Cohen, J. Biol. Chem., 1972:257:7609-11. European Patent Office publication number EP 177, 915 teaches the production of recombinant human EGF by E.coli transformed with a synthetic gene.

FGF has been isolated and purified from natural sources (R.R. Lobb, J.W. Harper, J.W. Fatt, Purification of Heparin-Binding growth factors, Anal. Biochem. 154:1-14, 1986). Production of recombinant FGF is described in published European patent application EP 248,819.

EP 200,341 describes the production of TGF-B by transforming a eukaryotic cell with a vector.

A process for obtaining TGF- is described in J.E. DeLarco and G.E. Todaro, Growth Factors from Murine Carcoma Virus-Transformed Cells, Pro. Natl. Acad. Sci. USA, 75:4001-4005, 1978.

Ep 219, 814 teaches the production of recombinant human IGF-I. E.Rinderknect and R.E. Humbel teach the production of IGF-I from natural sources in Polypeptides With the Non-Suppressible Insulin-Like and Cell-Growth Promoting Activities in Human Serum; Isolation, Chemical Characterization and some Biological Properties of Forms I aid II, Proc. Natl. Acad. Sci. USA, 73; 2365-2369, 1976.

As used herein, unless otherwise indicated, the term "growth factor" is intended to include the above described peptide growth factors, as well as biologically active fragments and derivatives thereof, including precursors that may be proteolytically processed into biologically active growth factors in corneal endothelial cells.

Cornea preservation solutions are well known. In general, those employed herein contain an aqueous nutrient and electrolyte solution, a glycosaminoglycan, a deturgescent agent(s), a buffer system(s), an energy source(s), an antioxidant(s), and membrane stabilizing components. Nutrient and electrolyte solutions are well defined in the art of tissue-culturing. Such solutions contain the essential nutrients and electrolytes at minimal concentrations necessary for cell maintenance and cell growth. The actual composition of the solutions may vary greatly. In general, they contain inorganic salts, such as calcium, magnesium, iron, sodium and potassium salts of carbonates, nitrates, phosphates, chloride and the like, essential and non-essential amino acids, vitamins and other essential nutrients.

Chemically defined basal nutrient media are commercially available, for example from Gibco Laboratories (3175 Stanley Road, Grand Island, New York 14073) and Microbiological Associates (P.O. Box 127, Briggs Ford Road, Walkersville, Maryland 21793) under the names Eagle's Minimal Essential Medium and TC199. Cornea] storage solutions have been adapted from these nutrient media. Commercially available corneal storage media useful in the present invention include CSM™ and Dexsol™ developed by R.L. Lindstrom, M.D. and Debra L. Skelnik, B.S., available from Chiron, Ophthalmics Inc. (Irvine, CA). Additional media useful in the present invention also include K-Sol™ corneal storage medium, a product of Coopervision Cilco (Bellevue, Washington 98008-5498) and McCarey-Kaufman (M-K™) medium available from Chiron, Ophthalmics, Inc. (Irvine, CA). Table I provides a detailed comparison of the media components of TC199, CSM™ ,Dexsol™, and K-Sol™ and M-K™.

## Description of the Preferred Embodiments

Preferred serumfree medical solutions for use in the composition and methods of this invention contain an aqueous electrolyte solution (e.g. Minimal Essential Medium and/or TC199), a glycosaminoglycan (e.g. standard or purified high or low molecular weight heparin sulfate, heparan sulfate, keratin sulfate, dermatin sulfate, chondroitin sulfate (A, B or C isomer) and/or sodium hyaluronate,) in a range of .01 mg/ml to 100 mg/ml; a deturgescent agent (e.g. low or high molecular weight polysaccharide, such as dextran, dextran sulfate, polyvinyl pyrrolidone, polyvinyl acetate, hydroxypropylmethyl cellulose, carboxypropylmethyl cellulose)in a range of .01 mg/ml to 100 mg/ml; an energy source and carbon source (e.g. glucose,pyruvate, fructose, dextrose)in a range of .05 mM to 10 mM; a buffer system (e.g. a bicarbonate buffer system and hydroxyethyl-piperizene ethanesulfonic acid, HEPES buffer) in a range of .1 mM to 100 mM; to maintain a physiologic pH (desirably between 6.8 and 7.6), an antioxidant (e.g.,ascorbic acid, 2-mercaptoethanol, glutathione, alpha tocopherol), in a range of .001 mM to 10 mM; membrane stabilizing agents (e.g. vitamins A and B, retinoic and/or cofactors, ethanolamine, and phosphoethanolamine, selenium and transferrin), in a range of .01 mg/ml to 500 mg/ml; and a growth factor (e.g. epidermal growth factor (EGF), insulin-like growth factor I (IGF-I), insulin-like growth factor II (IGF-II), acidic or basic growth factor (FGF), transforming growth factor alpha (TGF-$\alpha$), transforming growth factor beta (TGF-$\beta$), platelet derived growth factor (PDGF) and insulin) in a range of .001 ng/ml to 1 mg/ml.

The medical solution of this invention contains one or more growth factors in the amount sufficient to promote endothelial cell mitosis following low temperature eye bank storage. The excised corneas are aseptically transferred to containers of the corneal storage solution, which are then sealed. For storage and transport,these corneas are maintained at low temperature (e.g. 2°C to 15°C optimally at 4°C) to minimize the risk of bacterial growth and to reduce corneal tissue metabolic damage. It has been found that even at these low temperatures, the endothelial cells can be maintained for periods up to 14 days. The tissues are warmed to room temperature for at least one hour. Once the cornea is transplanted and the tissue warmed to 34°C to 37°C the endothelial (epithelial) cells have been found to mitose at a substantial rate, thus causing the endothelium (epithelium) to proliferate. In addition to providing a viable cornea for transplantation, the proliferation of cells provides greater wound healing. Various modifications can be made to the present invention without departing from the apparent scope thereof. For instance, the serumfree solution can be used in any medical application, and is not strictly limited to ophthalmology. The invention is further illustrated by the following examples, which is not intended to be limiting.

## Mode Of Operation

Example 1

Chondroitin Sulfate-Containing (CSM) Solution with Dextran and Insulin

To counteract the morphological and biochemical alterations encountered as a consequence of lower storage temperatures and reduced metabolic rates, insulin was added to the chondroitin sulfate-containing solution. Insulin has been demonstrated to increase the glucose uptake of cells by increasing the activity of glucose permease in the plasma membrane. Glucose permeases greatly increase the permeability of the cell membrane to glucose. Insulin's effect on glucose uptake and catabolism occurs within minutes and does not require new protein synthesis. Insulin was also added to the solution to help preserve the highly metabolic epithelial cell layer.

In addition to promoting greater glucose uptake, insulin can also act like a growth factor. The insulin-like growth factors (IGF) and insulin are closely related as shown, originally by amino acid sequence , and more recently by cDNA cloning and sequencing results. Insulin, IGF-I and IGF-II are capable of eliciting the same biological responses including cell multiplication. This suggests that they have similar or identical effector pathways.

On the cell surface there are two types of IGF receptors in addition to the well-described insulin receptor. Most cells have both types of IGF receptors as well as insulin receptors . IGF I, IGF II and insulin can interact with both insulin receptors and type I IGF receptors; only IGF I and IGF II interact with the type II receptor. Because of the extensive cross-reactivity of ligands with these three receptor types it is difficult to assign particular biological responses to specific receptors. Indeed, the requirements for high concentrations of insulin for the the growth of many cells in defined media suggest that the type I IGF receptor, rather than insulin, may be involved in the growth response. In vitro studies with insulin and human corneal endothelial cells demonstrate that insulin may enhance the mitogenic response of these cells when used at hyperphysiologic concentrations.

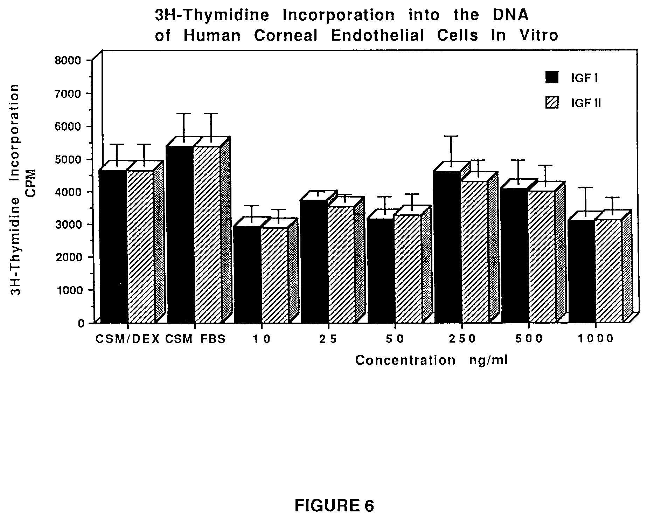

There are two types of insulin-like growth factor receptors (Figure 6). The type I receptor generally binds IGF-I more tightly than IGF-II and also interacts weakly with insulin. The type II receptor prefers IGF II over IGF I, and does not recognize insulin. There are many similarities between the type I IGF receptor and the insulin receptor; however, different ligand-binding properties, subtle differences in the sizes of the alpha and beta subunits, and immunoreactivity toward anti-receptor antibodies, allow us to distinguish between these two receptors. The similarities between the insulin receptor and the type I IGF receptor are striking, and suggest that, like insulin and IGF's, these two receptors may have evolved from a common ancestral gene. Therefore, at high concentrations of insulin, insulin may bind weakly to the IGF I receptor, and elicit a biological response such as cell division.

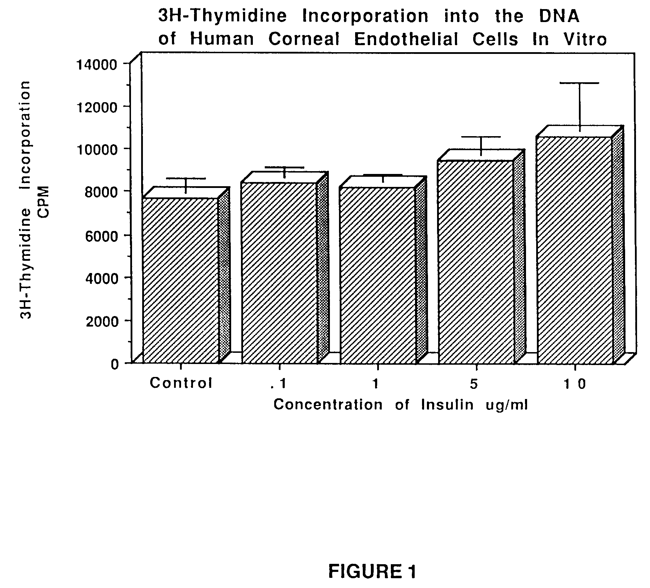

In order to determine the safety and efficacy of CSM supplemented with dextran and insulin, we first conducted an insulin concentration dose response curve on human corneal endothelial cells. A quantitative bioassay examining the rate of stimulation or inhibition of DNA synthesis of human corneal endothelial cells exposed to CSM supplemented with .1, 1,5 and 10 $\mu$g/ml was compared to CSM with no insulin (Figure 1) A prospective clinical trial was conducted evaluating corneal thickness and endothelial survival for corneas that had been stored in CSM solution supplemented with 1% dextran and 5 $\mu$g/ml of bovine insulin and transplanted into patients.

## Materials and Methods

### HCE Cell $^3$H-thymidine Incorporation Bioassay

Human corneal endothelial cells (third passage, donor age 35) were isolated and grown to confluency in 11 days, in the method previously described. The cells were then trypsinized, and resuspended in serum supplemented CSM. Ninety-six well tissue culture plates were seeded with 3 X $10^3$ cells/well in a final volume of 200 $\mu$l of CSM serum-supplemented medium. The third passage cells were maintained in a humidified incubator at 35.5°C in a 95% air : 5% CO2 atmosphere. After 24 hours of incubation in CSM medium supplemented with 10% fetal bovine serum, to permit attachment, the medium was removed, and each well was rinsed once with serum-free MEM with Earle's salts and 25 mM HEPES. The cells were then rinsed and incubated with CSM supplemented with 0,.1, 1, 5, and 10 $\mu$g/ml of bovine insulin (Sigma Chemical Company, St. Louis, MO) HCE cells were incubated in the presence of 1 microcurie/well of 3H-thymidine for 72 hours. Uptake was ended by the aspiration of the radioactive medium and rinsing the cells twice with serum-free MEM. HCE cells were detached with 0.5% trypsin and prepared for liquid scintillation counting. The $^3$H-thymidine counts (CPM) represent acid-insoluble counts. One-way analysis of variance and the Newman-Keuls multiple range test were used to evaluate statistical significance (p<0.05).

## Clinical Study

### Eye Bank Procedures

Human donor globes were immersed in 1.0% povidone iodine in normal saline for three minutes, followed by a one-minute immersion in normal saline. The globes were then rinsed with 12 cc of normal saline with a syringe fitted with a 18-gauge needle. Corneas from suitable donors were removed at the Minnesota Lion's Eye Bank an average of 13.2 hours after death, and placed in CSM medium supplemented with 1% dextran (40,000 mw) and 5$\mu$g/ml of bovine insulin (Sigma, St. Louis,M0) The medium was warmed to room temperature before the cornea was placed into it. The cornea was then cooled to 4°C and stored for an average of 3.8 days (range 1-9 days).

### Recipient Criteria

The following recipient diagnoses were considered for entry into the study: aphakic bullous keratopathy, Fuchs' dystrophy, pseudophakic bullous keratopathy, corneal scar, keratoconus, corneal ulcer, corneal decompensation and failed graft. The preoperative examination consisted of measurement of the best corrected visual acuity, intraocular pressure, slit lamp and funduscopic examination. Appropriate informed consent was obtained from each patient.

## Surgical Technique

Corneas were warmed to room temperature at the time of transplantation. The donor buttons were cut from the endothelial side with a corneal trephine press. Sodium hyaluronate (Healon) or sodium hyaluronate with chondroitin sulfate (Viscoat) was used in all cases. Operative and postoperative care was similar for all cases. Suturing techniques consisted of a combination of 12 interrupted 10-0 sutures with a running 11-0 nylon or mersilene suture. Gentamycin, Betamethasone and Ancef were injected subconjunctivally at the end of each procedure.

## Evaluation of Donor Endothelial Cell Count

Corneoscleral rims were stained with alizarin red S immediately after the central corneal button was cut. High power (250X) photographs of the endothelial monolayer were taken, and the corneal endothelial cell count was determined by counting four different fields from each photograph. Each photograph was of an area adjacent to the cut edge of the cornea. The grid area was calibrated by use of a microscopic micrometer prior to each photograph. Pre-operative endothelial cell density was 2534 ± 551 cells/mm2.

## Postoperative Treatment

Post-operatively all patients received neomycin or gentamycin drops four times daily during the first postoperative month. Topical steroids were administered as needed. Patients were evaluated during the first six months postoperatively for complications, rejection, corneal vascularization, infection, wound leak, dehiscence of wound, persistent epithelial defects, and overall corneal condition. Utrasonic pachymetry of the central cornea was performed at selected follow-up visits. With a contact, wide-field video microscope (Coopervision, Reading, CA) a recording of the central endothelium was made at 3 (3 ± 1 month), 6 (6 ± 2 months), and 12 (12 ± 4 months) months postoperatively. The total number of patients included in this study was 50. Percent cell loss was determined as the difference between the preoperative and postoperative cell density, divided by the preoperative cell density, multiplied by 100.

## Results

## Human Corneal Deturgescence Studies.

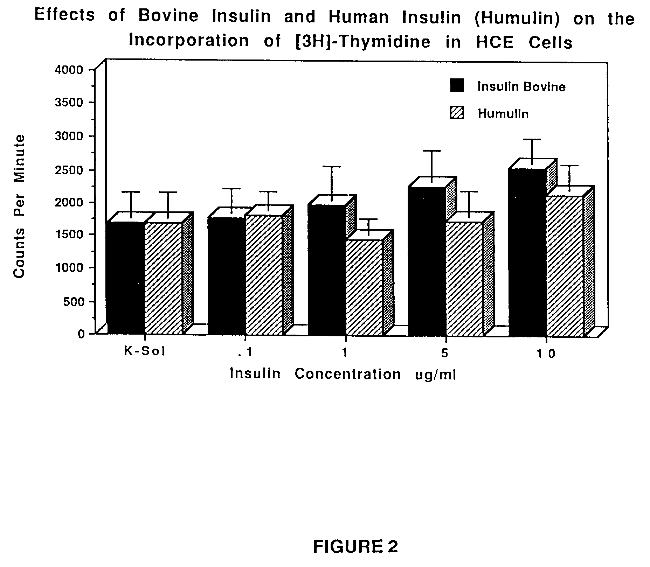

Corneal thickness measurements of human corneas stored at 4°C for 7 days in CSM-Dextran-Insulin medium and K-Sol medium are shown in Figure 2. Trefined corneal buttons placed in MEM after 7 days storage at 4°C increased in corneal thickness by 25.4% (CSM-Dextran-Insulin medium) and 41.6% (K-Sol medium) during the first thirty minutes No further increases in corneal thickness were noted after the first thirty minutes post-incubation.

## HCE Cell $^3$H-thymidine Incorporation Bioassay

Figure 1 shows the mean $^3$H-thymidine incorporation of human corneal endothelial cells incubated with CSM supplemented with .1, 1, 5, and 10 $\mu$g/ml of bovine insulin CSM. There was no significant difference in $^3$H-thymidine incorporation of HCE cells incubated with CSM (mean ± SD, 7713 ± 622 cpm) versus CSM with .1 $\mu$g/ml of insulin (8405 ± 488 cpm) and 1 $\mu$g/ml of insulin (8234 ± 355 cpm). However, HCE cells incubated with CSM containing 5 and 10 $\mu$g/ml insulin had significant increases in $^3$H-thymidine incorporation (5 $\mu$g/ml: 9511 ± 841 cpm; 10$\mu$g/ml: 10603 ± 2273 cpm) as compared to cells incubated with CSM alone (p<.05).

## Clinical Study

Three hundred and ninety-two corneas were transplanted utilizing CSM-Dextran-Insulin solution from January 1988 through June 1988. Several surgeons participated in the medium evaluation study and many of the patients were returned to their referring physicians post-procedure. Fifty patients were operated on by one surgeon (R. L. L.) and were included in the following study. The cornea donors had the following characteristics: donor age 46.8 ± 17.1, death to enucleation time 5.9 ± 6.6 hours, and death to preservation time 6.9 ± 4.9 hours. Storage time of corneas at 4°C was 3.8 ± 1.6 days. One-hundred percent of the CSM-Dextran-Insulin transplanted corneas were clear after 3 months. Mean percent endothelial cell increase at 3 and 6 months was +4.0 ± 15.8% and +8.4 ± 16.1% (Table 10). Corneal thickness was .534 ± .05 mm and .549 ±.05 mm, at 3 and 6 months, respectively. Post-operative intraocular pressures were within normal limits. No primary donor failures occurred in this CSM-Dextran-Insulin cornea group.

## Discussion

The morphological appearance of the corneal endothelium has been a major determinant of corneal suitability for keratoplasty. The functional status of the corneal endothelium has not been routinely utilized because of the interference of osmotic agents currently employed in corneal storage media. Temperature-reversal studies are considered to be a functional test for the viability of the corneal endothelium. Corneal hydration is a function of a dynamic balance between the constant imbibition pressure, ie. swelling pressure, of the stromal glycosaminoglycans and the metabolically active transport system, which is located in the the endothelium. In vitro corneal deturgescence studies with chondroitin sulfate-containing corneal storage media present a method to predict the degree of corneal hydration that will occur during surgery. Corneal thickness measurements on the whole, untrephined corneas often are not indicative of the full extent of corneal swelling encountered when the stromal glycosaminoglycans are exposed to the aqueous humor. Experimental results with human corneas demonstrate that changes in solution osmolality can result in substantial alterations in corneal thickness without marked changes in corneal endothelium. The osmotic hydration or dehydration that occurs in the cornea is thought to be due to the bulk flow of water through the intercellular spaces. The intercellular spaces are dilated, but the apical junctions remain attached.

The increased corneal thickening associated with CSM-stored corneas, with greater rebound swelling apparent at the time of surgery, was further reduced by the addition of 1% dextran and 5 $\mu$g/ml of insulin to CSM medium. No primary donor failures were noted with the CSM-Dextran-Insulin corneas, and endothelial cell preservation was significantly better than corneas stored in CSM medium alone. A moderate increase in mean endothelial cell density was observed at both 3 and 6 months post-operatively.

The safety of this CSM-Dextran-Insulin medium was demonstrated by in vitro studies with a human corneal endothelial cell model. Human corneal endothelial cells incubated with CSM medium supplemented with 5 and 10 $\mu$g/ml of insulin showed significant increases in DNA synthesis versus cells incubated with CSM medium alone, exhibiting a mitogenic effect. Therefore, the addition of insulin to chondroitin sulfate containing-medium did not adversely effect endothelial cell survival. Thus, this preliminary study supports the use of CSM with the addition of 1% dextran and 5 $\mu$g/ml of insulin, in order to reduce intraoperative swelling, to provide greater glucose uptake, and to potentiate cell growth.

Example 2

The Effects of Growth Factors on Corneal Preservation at 4°C

Epidermal growth factor (EGF) is a 53-residue polypeptide which was originally discovered in the submaxillary glands of male mice. This peptide was capable of inducing precocious eyelid opening and incisor eruption when injected into newborn mice. The receptor for EGF is widely distributed, and among the many cell types that carry the EGF receptor are human corneal endothelium and epithelium. In humans, EGF has been detected by radioimmunoassay in nearly all body fluids. Human EGF (hEGF), urogastrone, is the 53-amino acid homologue of murine EGF (mEGF) The sites of synthesis of EGF in humans are not clear.

The presence of EGF receptors on the corneal endothelium would suggest a potential capacity for cell division, and limited human corneal endothelial cell regeneration has been demonstrated in vivo after penetrating keratoplasty, and in response to injury. Normally, in the unwounded anterior chamber environment, the necessary growth factors needed to stimulate cell division may not be present or present in limiting amounts. It is possible that cell growth factor inhibitors may also be present in the anterior chamber, suppressing the entry of cells into the DNA synthesizing stage of the cell cycle.

In our most recent studies we have begun to investigate the potential value of EGF for inducing mitosis in human corneal endothelium after 4°C corneal storage. The importance of EGF in controlling normal cell growth has led to efforts directed at understanding EGF's mode of action. At the cellular level, EGF stimulates the proliferation of quiescent cells of many cell types . The biochemical mechanism of EGF altering or regulating the proliferative state of the cell is complex. The formation of EGF-receptor complexes on the cell surface initiates the first step in this mechanism. Under physiological conditions, the plasma membrane receptors for EGF are rapidly saturated (within 30-60 minutes). During this period of time two events occur in regard to EGF-receptor complexes and either, or both, may be involved in intracellular signalling.

First, the binding of EGF to the receptor activates the protein kinase that is intrinsic to the receptor. The EGF receptor, as deduced from the cDNA sequence, is a transmembrane protein that crosses the membrane once. The receptor can be depicted as having two domains. One part of the molecule protrudes from the membrane to form a a domain on the cell surface that contains the binding site for EGF and EGF-like ligands. The second portion of the receptor is located on the cytoplasmic side of the plasma membrane facing the intracellular environment. This cytoplasmic domain contains the tyrosine-specific protein kinase activity of the EGF receptor.

Co-ordinate with activation of protein kinase activity, EGF-receptor complexes are subject to a complex series of events that involve the formation of clusters on the cell surface and endocytic internalization of ligand and receptor. The binding of EGF to its receptor is followed by the accumulation of numerous EGF-EGF receptor complexes in discrete patches, or throughout the cell membrane. Ligand-receptor clustering is a prelude to EGF internalization and intracellular processing. EGF-EGF receptor complexes diffuse laterally in the cell membrane and are processed via clathrin-coated pits. The growth-promoting effects of EGF are accomplished by the binding of EGF to cellular receptors, which elicit the phosphorylation of proteins, which in turn transmit growth signals to the cell nucleus. The exact biochemistry of EGF-receptor processing is not adequately understood. EGF-receptors may be recycled, or internalized complexes may be terminally degraded in lysosomes. Therefore, membrane replacement of the EGF receptors must therefore come from preformed intracellular receptors, or from de novo synthesis.

The time course of $^{125}$I-hEGF to human fibroblasts demonstrates that maximal binding of hEGF occurs after 30-40 minutes of incubation at 37°C or 2.5 hours at 0°C. These results demonstrate that hEGF binds to cells at low temperature. The low temperature inhibits endocytosis. Further studies indicate that hEGF exists as an intact molecule primarily on the cell surface, and that both the internalization and degradation of of hEGF are temperature-dependent.

In the absence of ligand, effector receptors are presumably relatively stable plasma membrane populations. The endocytic uptake of these receptors can, however, be induced by ligand binding so that, for example, a half-maximal mitogenic dose of EGF can cause the internalization of up to 60% of its receptors within five minutes at 37°C .

The 4°C corneal storage system provides us with a unique system in which we can "load" the cornea with growth factors prior to transplantation. In 4°C corneal storage the stroma imbibes fluid from both the epithelial and endothelial surfaces, in addition to fluid flow at the cut edge. The cornea then becomes a sponge, absorbing approximately .5 cc of fluid during storage. EGF can also bind to receptors found on both the epithelium and endothelium . The bound EGF can then be internalized and promote a mitogenic effect once the cornea is warmed up to 34°C after transplantation. The intracellular EGF can then be slowly released during a 48-96 hour period post-keratoplasty.

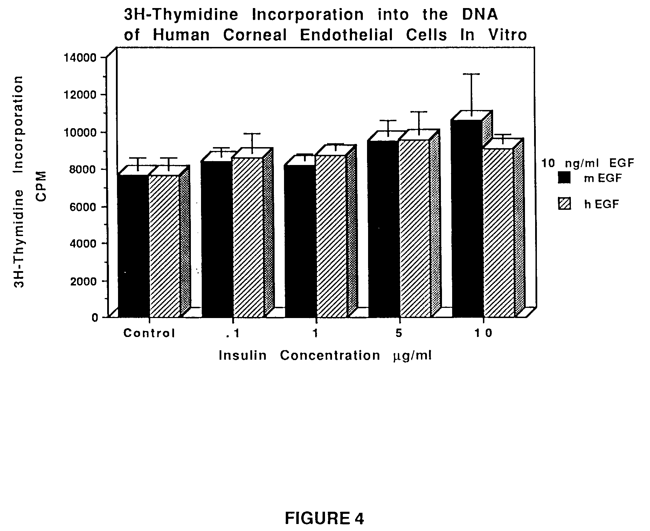

In our initial studies we used mEGF to determine the mitogenic effects of this growth factor on human corneal endothelium. Although mEGF arid hEGF elicit similar biological responses (Figure 4) it is clear that they have different amino acid compositions, with 70% homology occurring between the two polypeptides. The advent of recombinant DNA techniques has made possible the mass production of hEGF, and provided a unique opportunity to examine the mitogenic properties of a human polypeptide upon human cells in vitro.

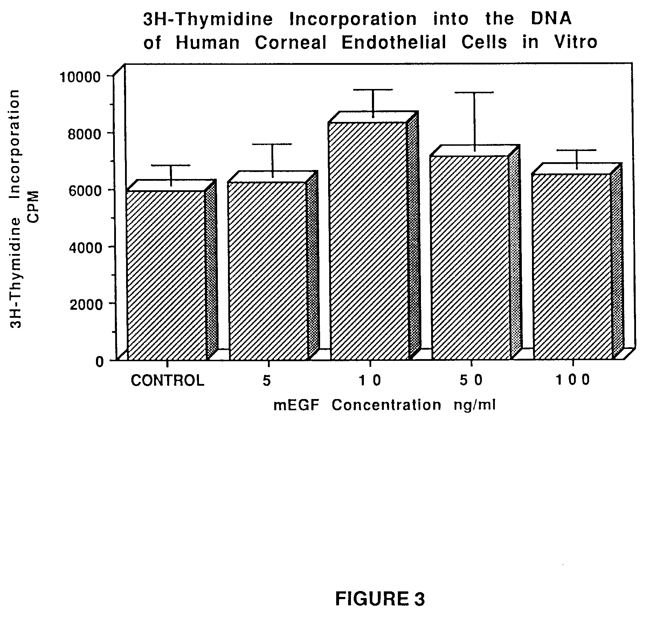

In order to determine the safety and efficacy of CSM supplemented with dextran insulin and EGF, we first conducted an EGF concentration dose response curve on human corneal endothelial cells. A quantitative bioassay examining the rate of stimulation or inhibition of DNA synthesis of human corneal endothelial cells exposed to CSM supplemented with 5,10, 50 and 100 ng/ml EGF was compared to CSM with no EGF (Figure 3). To study the synergistic effect of EGF and insulin on DNA synthesis we compared CSM with 10 ng/ml EGF with 0,.1, 1, 5, and 10 $\mu$g/ml insulin. In vitro studies analyzing the length of storage time and the ability to support corneal endothelial cell viability were also conducted using human corneas stored for 7, 10 and 14 days at 4°C in CSM-Dextran-Insulin solution or CSM-Dextran-Insulin-EGF solution Next a prospective clinical trial was conducted evaluating corneal thickness and endothelial survival for corneas that had been stored in CSM solution supplemented with 1% dextran, 5$\mu$g/ml of bovine insulin, and 10 ng/ml of mEGF and transplanted into patients.

Materials and Methods

HCE Cell $^3$H-thymidine Incorporation Bioassay

Human corneal endothelial cells (third passage, donor age 28) were isolated and grown to confluency in 9 days. The cells were then trypsinized, and resuspended in serum supplemented CSM. Ninety-six well tissue culture plates were seeded with 3 X 10$^3$ cells/well in a final volume of 200 $\mu$l of CSM serum-supplemented solution. The third passage cells were maintained in a humidified incubator at 35.5°C in a 95% air : 5% CO2 atmosphere. After 24 hours of incubation in CSM solution supplemented with 10% fetal bovine serum, to permit attachment, the medium was removed, and each well was rinsed once with serum-free MEM with Earle's salts and 25 mM HEPES.

EGF Dose Response Curve: The cells were then rinsed and incubated with CSM supplemented with 0, 5,10, 50 and 100 ng/ml of mEGF (Collaborative Research, Lexington,MA) (Figure 3).

Insulin Dose Response Curve with mEGF and hEGF: HCE cells were incubated with CSM containing 10 ng/ml of mEGF or hEGF supplemented with 0, .1, 1, 5 and 10 $\mu$g/ml of bovine insulin (Figure 4).

Insulin (Bovine and Human) Dose Response Curve with mEGF: HCE cells were incubated with CSM containing 10 ng/ml of mEGF supplemented with 0, .1, 1, 5 and 10 $\mu$g/ml of bovine or human insulin (Figure 2).

HCE cells were incubated in the presence of 1 microcurie/well of ³H-thymidine for 72 hours. Uptake was ended by the aspiration of the radioactive medium and rinsing the cells twice with serum-free MEM. HCE cells were detached with 0.5% trypsin and prepared for liquid scintillation counting. The ³H-thymidine counts (cpm) represent acid-insoluble counts. One-way analysis of variance and the Newman-Keuls multiple range test were used to evaluate statistical significance (p<0.05).

In Vitro Evaluation of Human Corneas Stored at 4°C

Human donor globes were immersed in 1.0% povidone iodine in normal saline for three minutes, followed by a one-minute immersion in normal saline. The globes were then rinsed with 12 cc of normal saline with a syringe fitted with a 18-gauge needle. Nine paired corneas from donors unsuitable for transplantation because of age or cause of death were removed at the Minnesota Lion's Eye Bank an average of 12.5 hours after death, and placed in 20 ml of CSM solution supplemented 1% dextran (40,000 mw) and 5$\mu$g/ml of bovine insulin (Sigma, St. Louis, MO) or CSM supplemented with 1% dextran (40,000 mw), 5$\mu$g/ml of bovine insulin and 10 ng/ml of mEGF (Collaborative Research, Lexington,MA). The solution was warmed to room temperature before the cornea was placed into it, and allowed to remain at room temperature for 30 minutes. The cornea was then cooled to 4°C. Three paired corneas were stored in each medium for three different time intervals: 7, 10 and 14 days. The corneas were allowed to warm up for two hours at 34°C, and were evaluated in a masked fashion following removal from numbered bottles. Endothelial cell viability was determined after staining the cornea with 1% trypan blue, whereas membrane integrity and cell borders were assessed after staining with alizarin red S. Central endothelial cell photographs were taken at high (250X) and low power (100X).

Clinical Study

Eye Bank Procedures

Human donor globes were immersed in 1.0% povidone iodine in normal saline for three minutes, followed by a one-minute immersion in normal saline. The globes were then rinsed with 12 cc of normal saline with a syringe fitted with a 18-gauge needle. Corneas from suitable donors were removed at the Minnesota Lion's Eye Bank an average of 16.6 hours after death, and placed in CSM solution supplemented with 1% dextran (40,000 mw), 5$\mu$g/ml of bovine insulin (Sigma, St. Louis,M0)and 10 ng/ml of mEGF (Collaborative Research, Lexington,MA). The solution was warmed to room temperature before the cornea was placed into it The cornea remained at room temperature for 30 minutes, cooled to 4°C, and stored for an average of 4.2 days (range 1-9 days).

Recipient Criteria

The following recipient diagnoses were considered for entry into the study: aphakic bullous keratopathy, Fuchs' dystrophy, pseudophakic bullous keratopathy, corneal scar, keratoconus, corneal ulcer, corneal decompensation and failed graft. The preoperative examination consisted of measurement of the best corrected visual acuity, intraocular pressure, slit lamp and funduscopic examination. Appropriate informed consent was obtained from each patient.

Surgical Technique

Corneas were warmed to room temperature (1-2 hours) prior to transplantation. The donor buttons were cut from the endothelial side with a corneal trephine press. Sodium hyaluronate (Healon) or sodium hyaluronate with chondroitin sulfate (Viscoat) was used in all cases. Operative and postoperative care was similar for all cases. Suturing techniques consisted of a combination of 12 interrupted 10-0 sutures with a running 11-0 nylon or mersilene suture. Gentamycin, Betamethasone and Ancef were injected subconjunctivally at the end of each procedure.

Evaluation of Donor Endothelial Cell Count

Corneoscleral rims were stained with alizarin red S immediately after the central corneal button was cut. High power (250X) photographs of the endothelial monolayer were taken, and the corneal endothelial cell count was determined by counting four different fields from each photograph. Each photograph was of an area adjacent to the cut edge of the cornea. The grid area was calibrated by use of a microscopic micrometer prior to each photograph. Pre-operative endothelial cell density was 2201± 570 cells/mm2.

Postoperative Treatment

Post-operatively all patients received neomycin or gentamycin drops four times daily during the first postoperative month. Topical steroids were administered as needed. Patients were evaluated during the first six months postoperatively for complications, rejection, corneal vascularization, infection, wound leak, dehiscence of wound, persistent epithelial defects, and overall corneal condition. Utrasonic pachymetry of the central cornea was performed at selected follow-up visits. With a contact, wide-field video microsope (Coopervision, Reading, CA) a recording of the central endothelial was made at 3 (3 ± 1 month), 6 (6 ± 2 months), and 12 (12 ± 4 months) months postoperatively. The total number of patients included in this study was 42. Percent cell loss was determined as the difference between the preoperative and postoperative cell density, divided by the preoperative cell density, multiplied by 100.

Results

HCE Cell $^3$H-thymidine Incorporation Bioassay

Figure 3 shows the mean 3H-thymidine incorporation of human corneal endothelial cells incubated with CSM supplemented with 0, 5, 10, 50 and 100 ng/ml of mEGF. There was no significant difference in $^3$H-thymidine incorporation of HCE incubated with CSM (mean ± SD, 5957 ± 676 cpm) versus CSM with 5 ng/ml mEGF (6260 ± 1131 cpm), or CSM with 100 ng/ml mEGF (6503 ± 628 cpm). Significant increases in CSM with 10 ng/ml mEGF (8351 ± 933 cpm) and CSM with 50 ng/ml mEGF (7162 ± 2018 cpm) were demonstrated as compared to CSM alone.

HCE cells incubated with CSM containing 10 ng/ml of mEGF or hEGF supplemented with .1 and 1μg/ml of bovine insulin demonstrated no significant difference in $^3$H-thymidine incorporation than the EGF controls without insulin. HCE cells incubated with CSM supplemented with 5 and 10 μg/ml of bovine insulin with either mEGF or hEGF exhibited statistically significant increases over the EGF controls without insulin (Figure 4).

Furthermore, HCE cells incubated with CSM containing 10 ng/ml of mEGF, supplemented with .1 and 1 μg/ml of either bovine insulin or human insulin, demonstrated no significant difference in $^3$H-thymidine incorporation than the EGF control without insulin. HCE cells incubated with CSM supplemented with 5 and 10 μg/ml of bovine insulin or human insulin with mEGF exhibited statistically significant increases over the EGF controls without insulin (Figure 2).

In Vitro Evaluation of Human Corneas Stored at 4°C

Microscopic evaluation of epithelial cell layers of corneas stored in both CSM-Dextran-Insulin and CSM-Dextran-Insulin-EGF appeared loosely adherent with minimal sloughing of the epithelium at 7 and 10 days storage at 4°C. At 14 days storage for both media, the basal epithelium was still present but was edematous, and rounded.

Corneas stored in both CSM-Dextran-Insulin and CSM-Dextran-Insulin-EGF maintained endothelial cell membrane integrity, with distinctly stained borders at 7,10 and 14 days storage at 4°C.

Clinical Study

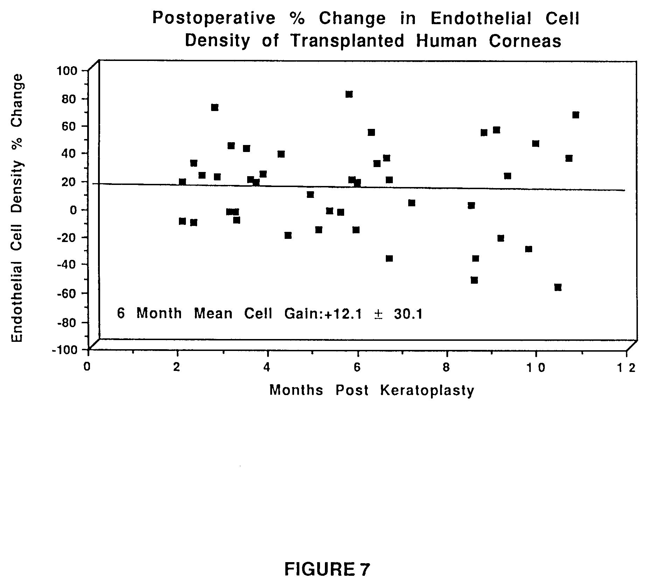

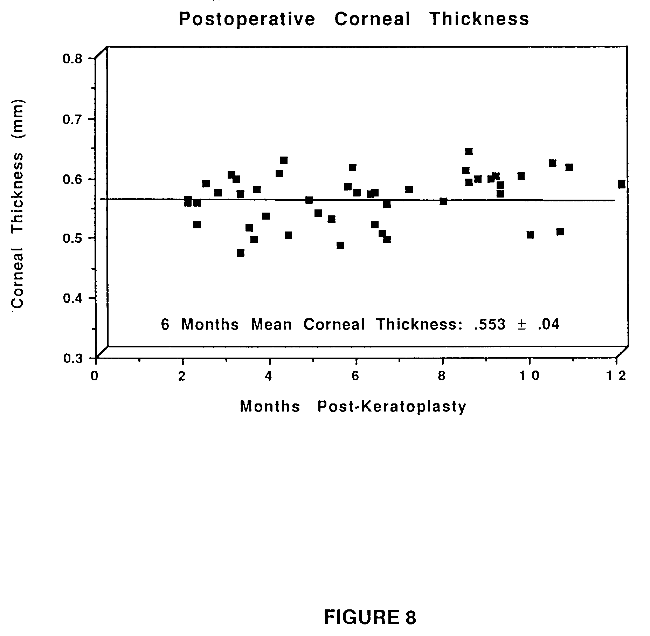

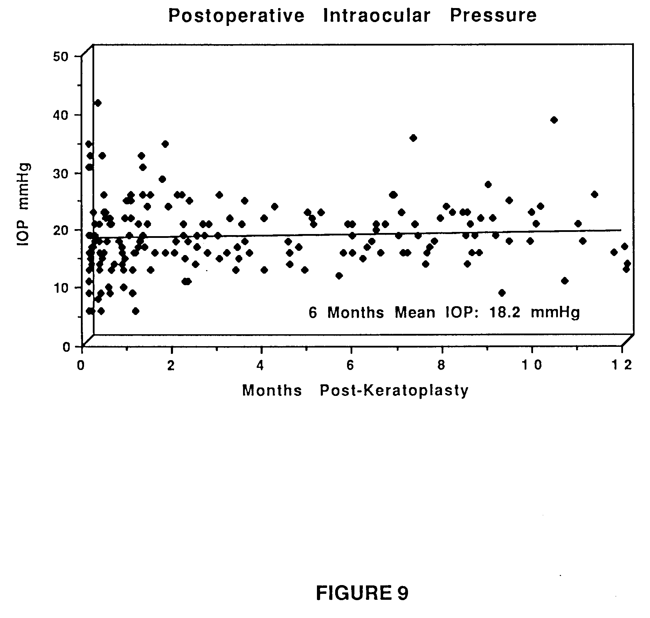

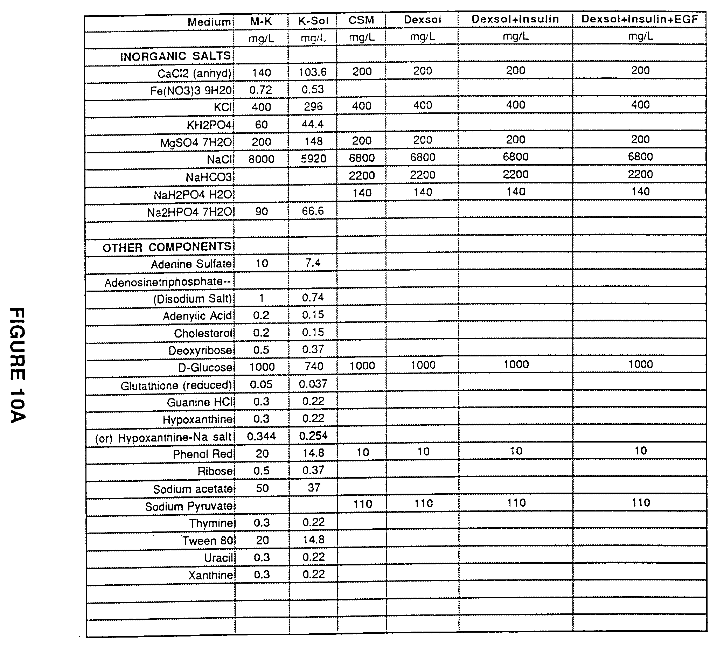

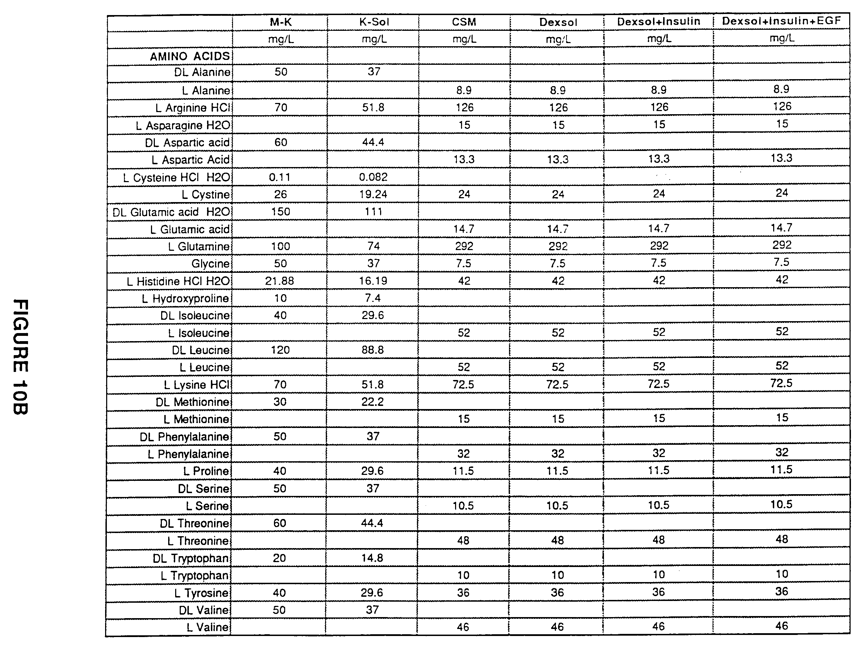

Forty-two corneas were transplanted utilizing CSM-Dextran-Insulin-EGF solution from June 1987 through December 1987. Forty-two patients were operated on by one surgeon (R. L. L.) and were included in the following study. The cornea donors had the following characteristics: donor age 40.4 ± 18.2, death to enucleation time 7.7 ± 4.8 hours, and death to preservation time 8.9 ± 5.2 hours. Storage time of corneas at 4°C was 4.2 ± 1.8 days. One-hundred percent of the CSM-Dextran-Insulin-EGF transplanted corneas were clear after 3 months. Mean percent endothelial cell gain at 3, 6 and 12 months (Figure 7) was +13.0 ± 23.6%, +12.1 ± 30.1% and +10.3 ± 33.5% (Figure 10). Corneal thickness was .554.± .04 mm, .553 ±.04 mm, and .553 ± .04 at 3, 6 and 12 months, respectively (Figure 8). Post-operative intraocular pressures were within normal limits (Figure 9). No primary donor failures occurred in this CSM-Dextran-Insulin-EGF cornea group.

Discussion

In this preliminary clinical study utilizing corneas stored in CSM-Dextran-Insulin-EGF solution, we have begun to investigate the potential value of use of EGF for inducing mitosis in human corneal endothelium after 4°C corneal storage. No primary donor failures were noted with the CSM-Dextran-Insulin-EGF corneas, and endothelial cell preservation was significantly better than with corneas stored in CSM solution alone. A increase in mean endothelial cell density was observed at 3, 6 and 12 months post-operatively. Normal corneal thickness and intraocular pressures were maintained throughout the post-operative follow-up period.

In the 4°C corneal storage system the cornea can be preloaded with growth factors prior to transplantation. After transplantation these growth factors can be slowly released into the anterior chamber and provide a prolonged contact with the endothelium. During the first few days postkeratoplasty there is a good opportunity for cells to be stimulated to undergo mitosis.

A critical period in the life of the cell occurs immediately after each mitosis a threshold is reached as to whether or not to remain within the cell cycle. During early development cells opt to remain in the cell cycle, but as cell numbers increase groups of cells stop growing and enter a GO phase, during which they differentiate to perform some specific function. Many cells are terminally differentiated and are unable to under go mitosis. However, there are many differentiated cells that retain the ability to return to the cell cycle if provided with an appropriate mitogenic signal, usually in the form of specific growth factor. We feel that growth factors such as EGF and insulin may provides the stimulus to promote limited endothelial cell mitosis. Therefore, the slow release of growth factor from the cornea over a period of days may provide the continuous stimulation needed to commit these endothelial cells to undergo limited cell division.

The safety of this CSM-Dextran-Insulin-EGF solution was demonstrated by in vitro studies with a human corneal endothelial cell model. Effective concentrations of EGF and insulin were determined utilizing a human corneal endothelial cell $^3$H-thymidine incorporation bioassay. A optical mitogenic effect was exhibited utilizing 10 ng/ml of mEGF in combination with 5 $\mu$g/ml of bovine insulin. In addition recombinant hEGF and human insulin were similar in their mitogenic properties in relation to the stimulation of DNA synthesis of human corneal endothelial cells. In vitro studies of human corneas stored in CSM-Dextran-Insulin-EGF solution for 7,10 and 14 days maintained a normal intact endothelial cell monolayer. Therefore, the addition of EGF to chondroitin sulfate containing-solution did not adversely effect endothelial cell survival. Thus, this preliminary study supports the use of CSM with the addition of 1% dextran, 5 $\mu$g/ml of insulin and 10 ng/ml of EGF, in order to reduce intraoperative swelling, to provide greater glucose uptake, and to enhance the mitotic potential of endothelial cells.

Preferred solutions of the present invention have one or more of the following features:
- the growth factor is polypeptide
- the growth factor is recombinant
- the growth factor is derived from a natural biological source
- the growth factor is one or more of epidermal growth factor (EGF),acidic or basic fibroblastic growth factor (FGF), transforming growth factor alpha (TGF-$\alpha$), transforming growth factor beta (TGF - $\beta$), insulin-like growth factor I(IGF-I) and insulin-like growth factor II (IGF-II) and insulin
- the growth factor is insulin
- the growth factor is insulin at a concentration of about 0.001 ng/ml to 1 mg/ml
- the growth factor is insulin at a concentration of about 10 ug/ml

- the growth factor is human recombinant insulin at a concentration of about 10 ug/ml
- the growth factor is EGF
- the growth factor is EGF at a concentration of about 0.001 ng/ml to 1 mg/ml
- the growth factor is EGF at a concentration of about 10 ng/ml
- the growth factor is human recombinant EGF at a concentration of about 10 ng/ml
- the growth factors are insulin and EGF
- the growth factors are insulin and EGF at a concentration of about 0.001 ng/ml to 1mg/ml
- the growth factors are insulin at a concentration of about 10 ug/ml and EGF is at a concentration of about 10 ng/ml
- the growth factor is human recombinant insulin at a concentration of about 10 ug/ml and human recombinant EGF is at a concentration of about 10 ng/ml
- the base medium comprises Eagle's, minimal essential medium (MEM)
- the base medium comprises TC 199

In the methods of the present invention the temperature said to be "about 34°C" can for example be in the range 33 to 35°C.

**Claims**

1. A serum-free medical solution characterised in that it comprises:
   a. an aqueous nutrient and electrolyte solution;
   b. a glycosaminoglycan;
   c. a deturgescent agent;
   d. a buffer system;
   e. an energy source;
   f. an antioxidant; and
   g. a growth factor.

2. A serum-free medical solution characterised in that it contains at least one or more growth factors which maintain and enhance the preservation of eye tissues, including human corneal tissues at low temperatures (2°C to 15°C) with a physiological pH (6.8 to 7.8), comprising:
   a. an aqueous nutrient and electrolyte solution;
   b. a glycosaminoglycan;
   c. a deturgescent agent;
   d. a buffer system;
   e. an energy source;
   f. an antioxidant;
   g. a membrane stabilizing component;
   h. an anitbiotic and or antimycotic; and,
   i. a growth factor.

3. A solution suitable for preserving cornea,which contains one or more growth factors.

4. A serumfree solution which contains one or more growth factors which maintain and enhance the preservation of eye tissues, (e.g.human corneal tissues) at low temperatures (e.g. 2°C to 15°C) with a physiological pH , comprising:

A. An aqueous nutrient and electrolyte solution selected from the group of:
    1. Eagle's minimal essential medium (MEM)
    2. TC199 medium
    3. A combination of Eagle's minimal essential medium (MEM) and TC199 ;

B. A glycosaminoglycan in the range of .01 mg/ml to 100 mg/ml selected from the group of:
    1. Chondroitin sulfate
    2. Dermatin sulfate
    3. Heparin sulfate
    4. Keratin sulfate
    5. Hyaluronic acid ;

C. A deturgescent agent in the range of .01 mg/ml to 100 mg/ml selected from the group of:
    1. Dextran
    2. Dextran sulfate
    3. Polyvinyl pyrrolidone
    4. Polyvinyl acetate
    5. Hydroxypropylmethyl cellulose
    6. Carboxypropylmethyl cellulose ;

D. A buffer system in a range of .1 mM to 100 mM selected from the group of:
    1. Bicarbonate buffer
    2. HEPES buffer ;

E. An energy source in a range of .05 mM to 10 mM selected from the group of:
    1. Glucose
    2. Pyruvate
    3. Fructose
    4. Dextrose ;

F. An antioxidant in a range of .001 mM to 10 mM selected from the group of:
    1. Ascorbic Acid
    2. 2-mercaptoethanol
    3. Glutathione
    4. Alpha-Tocopherol ;

G. A membrane stabilizing component in a range of .01 mg/ml to 500 mg/ml selected from the group of:
    1.Vitamins A
    2. Vitamin B
    3. Retinoic acid
    4. Ethanolamine
    5. Phosphoethanolamine
    6. Selenium
    7. Transferrin ;

H. An antibiotic and or antimycotic in the range of .1 ug/ml to 1 mg/ml selected from the group of:
    1. Gentamycin
    2. Fungizone ;

H. A growth factor in a range of .001 ng/ml to 1 mg/ml selected from the group of:
    1. Epidermal growth factor (EGF)
    2. Insulin-like growth factor I (IGF-I)
    3. Insulin-like growth factor II (IGF-II)
    4. Acidic or basic fibroblastic growth factor (FGF)
    5. Transforming growth factor alpha (TGF-$\alpha$)
    6. Transforming growth factor beta (TGF-$B$)
    7. Platelet derived growth factor (PDGF)
    8. Insulin.

5. A solution according to Claim 2 or 4, wherein the preservation step is conducted at about 2°C to about 15°C.

6. A method of enhancing the quality of eye tissues prior to keratoplasty, comprising storing at about 2°C to 15°C said tissues in a serum-free solution containing one or more growth factors and warming said tissues to about 34°C.

7. A method according to Claim 6, wherein said growth factors are one or more selected from the group of:
    1. Epidermal growth factor (EGF)
    2. Insulin-like growth factor I (IGF-1)
    3. Insulin-like growth factor II (IGF-II)
    4. Acidic or basic fibroblastic growth factor (FGF)
    5. Transforming growth factor alpha (TGF-$\alpha$)
    6. Transforming growth factor beta (TGF-$\beta$)
    7. Platelet derived growth factor (PDGF)
    8. Insulin.

8. A method of enhancing the quality of eye tissues prior to keratoplasty comprising storing at about 2°C to 15°C said tissues in a serum-free solution containing or more more growth factors and warming said tissues to about 34°C.

9. A method according to Claim 8, wherein said growth factor or factors are each in the range of 0.001 ng/ml to 1 mg/ml and selected from the group of:
    1. Epidermal growth factor (EGF)
    2. Insulin-like growth factor I (IGF-I)

3. Insulin-like growth factor II (IGF-II)

4. Acidic or basic fibroblastic growth factor FGF

5. Transforming growth factor alpha (TGF-$\alpha$)

6. Transforming growth factor beta (TGF-$B$)

7. Platelet derived growth factor (PDGF)

8. Insulin .

10. In a penetrating keratoplasty procedure comprising removing donor corneal tissues, storing said tissues and then implanting said tissues in a recipient patient, the improvement comprising storing said tissues at about 2°C to 15°C in serumfree solution comprising one or more growth factors.

**FIGURE 1**

Effects of Bovine Insulin and Human Insulin (Humulin) on the Incorporation of [3H]-Thymidine in HCE Cells

FIGURE 2

3H-Thymidine Incorporation into the DNA of Human Corneal Endothelial Cells in Vitro

FIGURE 3

3H-Thymidine Incorporation into the DNA of Human Corneal Endothelial Cells In Vitro

**FIGURE 4**

18

3H-Thymidine Incorporation into the DNA
of Human Corneal Endothelial Cells In Vitro

FIGURE 5

19

3H-Thymidine Incorporation into the DNA of Human Corneal Endothelial Cells In Vitro

FIGURE 6

Postoperative % Change in Endothelial Cell Density of Transplanted Human Corneas

FIGURE 7

FIGURE 8

Postoperative Intraocular Pressure

6 Months Mean IOP: 18.2 mmHg

**FIGURE 9**

| Medium | M-K mg/L | K-Sol mg/L | CSM mg/L | Dexsol mg/L | Dexsol+Insulin mg/L | Dexsol+Insulin+EGF mg/L |
|---|---|---|---|---|---|---|
| **INORGANIC SALTS** | | | | | | |
| CaCl2 (anhyd) | 140 | 103.6 | 200 | 200 | 200 | 200 |
| Fe(NO3)3 9H2O | 0.72 | 0.53 | | | | |
| KCl | 400 | 296 | 400 | 400 | 400 | 400 |
| KH2PO4 | 60 | 44.4 | | | | |
| MgSO4 7H2O | 200 | 148 | 200 | 200 | 200 | 200 |
| NaCl | 8000 | 5920 | 6800 | 6800 | 6800 | 6800 |
| NaHCO3 | | | 2200 | 2200 | 2200 | 2200 |
| NaH2PO4 H2O | | | 140 | 140 | 140 | 140 |
| Na2HPO4 7H2O | 90 | 66.6 | | | | |
| | | | | | | |
| **OTHER COMPONENTS** | | | | | | |
| Adenine Sulfate | 10 | 7.4 | | | | |
| Adenosinetriphosphate-- (Disodium Salt) | 1 | 0.74 | | | | |
| Adenylic Acid | 0.2 | 0.15 | | | | |
| Cholesterol | 0.2 | 0.15 | | | | |
| Deoxyribose | 0.5 | 0.37 | | | | |
| D-Glucose | 1000 | 740 | 1000 | 1000 | 1000 | 1000 |
| Glutathione (reduced) | 0.05 | 0.037 | | | | |
| Guanine HCl | 0.3 | 0.22 | | | | |
| Hypoxanthine | 0.3 | 0.22 | | | | |
| (or) Hypoxanthine-Na salt | 0.344 | 0.254 | | | | |
| Phenol Red | 20 | 14.8 | 10 | 10 | 10 | 10 |
| Ribose | 0.5 | 0.37 | | | | |
| Sodium acetate | 50 | 37 | | | | |
| Sodium Pyruvate | | | 110 | 110 | 110 | 110 |
| Thymine | 0.3 | 0.22 | | | | |
| Tween 80 | 20 | 14.8 | | | | |
| Uracil | 0.3 | 0.22 | | | | |
| Xanthine | 0.3 | 0.22 | | | | |

**FIGURE 10A**

| AMINO ACIDS | M-K mg/L | K-Sol mg/L | CSM mg/L | Dexsol mg/L | Dexsol+Insulin mg/L | Dexsol+Insulin+EGF mg/L |
|---|---|---|---|---|---|---|
| DL Alanine | 50 | 37 | | | | |
| L Alanine | | | 8.9 | 8.9 | 8.9 | 8.9 |
| L Arginine HCl | 70 | 51.8 | 126 | 126 | 126 | 126 |
| L Asparagine H2O | | | 15 | 15 | 15 | 15 |
| DL Aspartic acid | 60 | 44.4 | | | | |
| L Aspartic Acid | | | 13.3 | 13.3 | 13.3 | 13.3 |
| L Cysteine HCl H2O | 0.11 | 0.082 | | | | |
| L Cystine | 26 | 19.24 | 24 | 24 | 24 | 24 |
| DL Glutamic acid H2O | 150 | 111 | | | | |
| L Glutamic acid | | | 14.7 | 14.7 | 14.7 | 14.7 |
| L Glutamine | 100 | 74 | 292 | 292 | 292 | 292 |
| Glycine | 50 | 37 | 7.5 | 7.5 | 7.5 | 7.5 |
| L Histidine HCl H2O | 21.88 | 16.19 | 42 | 42 | 42 | 42 |
| L Hydroxyproline | 10 | 7.4 | | | | |
| DL Isoleucine | 40 | 29.6 | | | | |
| L Isoleucine | | | 52 | 52 | 52 | 52 |
| DL Leucine | 120 | 88.8 | | | | |
| L Leucine | | | 52 | 52 | 52 | 52 |
| L Lysine HCl | 70 | 51.8 | 72.5 | 72.5 | 72.5 | 72.5 |
| DL Methionine | 30 | 22.2 | | | | |
| L Methionine | | | 15 | 15 | 15 | 15 |
| DL Phenylalanine | 50 | 37 | | | | |
| L Phenylalanine | | | 32 | 32 | 32 | 32 |
| L Proline | 40 | 29.6 | 11.5 | 11.5 | 11.5 | 11.5 |
| DL Serine | 50 | 37 | | | | |
| L Serine | | | 10.5 | 10.5 | 10.5 | 10.5 |
| DL Threonine | 60 | 44.4 | | | | |
| L Threonine | | | 48 | 48 | 48 | 48 |
| DL Tryptophan | 20 | 14.8 | | | | |
| L Tryptophan | | | 10 | 10 | 10 | 10 |
| L Tyrosine | 40 | 29.6 | 36 | 36 | 36 | 36 |
| DL Valine | 50 | 37 | | | | |
| L Valine | | | 46 | 46 | 46 | 46 |

**FIGURE 10B**

FIGURE 10C

EP 0 516 901 A1

|  | M-K | K-Sol | CSM | Dexsol | Dexsol+Insulin | Dexsol+Insulin+EGF |
|---|---|---|---|---|---|---|
|  | mg/L | mg/L | mg/L | mg/L | mg/L | mg/L |
| **VITAMINS** |  |  |  |  |  |  |
| Ascorbic acid | 0.05 | 0.037 |  |  |  |  |
| Alpha Tocopherol Phosphate | 0.01 | 0.0074 |  |  |  |  |
| (Disodium salt) |  |  |  |  |  |  |
| d-Biotin | 0.01 | 0.0074 |  |  |  |  |
| Calciferol | 0.1 | 0.074 |  |  |  |  |
| D-Ca pantothenate | 0.01 | 0.0074 | 1 | 1 | 1 | 1 |
| Choline Chloride | 0.5 | 0.037 | 1 | 1 | 1 | 1 |
| Folic acid | 0.01 | 0.0074 | 1 | 1 | 1 | 1 |
| i-Inositol | 0.05 | 0.037 | 2 | 2 | 2 | 2 |
| Menadione | 0.01 | 0.0074 |  |  |  |  |
| Niacin | 0.025 | 0.019 |  |  |  |  |
| Niacinamide | 0.025 | 0.019 |  |  |  |  |
| Nicotinamide |  |  | 1 | 1 | 1 | 1 |
| Para-aminobenzoic acid | 0.05 | 0.037 |  |  |  |  |
| Pyridoxal HCl | 0.025 | 0.019 | 1 | 1 | 1 | 1 |
| Pyridoxine HCl | 0.025 | 0.019 |  |  |  |  |
| Riboflavin | 0.01 | 0.0074 | 0.1 | 0.1 | 0.1 | 0.1 |
| Thiamine HCl | 0.01 | 0.0074 | 1 | 1 | 1 | 1 |
| Vitamin A (acetate) | 0.014 | 0.0104 |  |  |  |  |
|  |  |  |  |  |  |  |
| **Additional Components** |  |  |  |  |  |  |
| Chondroitin Sulfate % |  | 2.5 | 1.35 | 1.35 | 1.35 | 1.35 |
| Gentamycin µg/ml |  | 100 | 100 | 100 | 100 | 100 |
| Dextran (45,000 MW) % | 5.00 |  |  | 1.00 | 1.00 | 1.00 |
| Insulin µg/ml |  |  |  |  | 5.00 | 5.00 |
| EGF ng/ml |  |  |  |  |  | 10.00 |

Table 2

EP 0 516 901 A1

FIGURE 11

| Donor Cornea Information | Age of Donor Years | PM Time Hours | PE Time Hours | Days Storage | Number of Corneas Tx |
|---|---|---|---|---|---|
| CSM-Dextran | 44.9 ± 16.4 | 5.7 + 3.2 | 5.9 ± 4.6 | 4.3 ± 1.7 | 63 |
| CSM-Dextran-Insulin | 46.8 ± 17.1 | 5.9 ± 6.6 | 6.9 ± 4.9 | 3.8 ± 1.6 | 50 |
| CSM-Dextran-Insulin-EGF | 40.4 ± 18.2 | 7.7 ± 4.8 | 8.9 ± 5.2 | 4.2 ± 1.8 | 42 |

| | Post-Keratoplasty | | |
|---|---|---|---|
| Endothelial Cell Density % Change | 3 Months | 6 Months | 12 months |
| CSM-Dextran | -9.2 ± 20.9 | -10.9 ± 21.9 | -14.6 ± 15.5 |
| CSM-Dextran-Insulin | +4.0 ± 15.8 | +8.4 ± 16.1 | |
| CSM-Dextran-Insulin-EGF | +13.0 ± 23.6 | +12.1 ± 30.1 | +10.3 ± 33.5 |

| Postoperative Corneal Thickness mm | | | |
|---|---|---|---|
| CSM-Dextran | .533 ± .06 | .532 ± .06 | .525 ± .05 |
| CSM-Dextran-Insulin | .534 ±.05 | .549 ± .05 | |
| CSM-Dextran-Insulin-EGF | .554 ± .04 | .553 ±.04 | .553 ± .04 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | US-A-5 013 714 (LINDSTROM R.L. ET AL)<br>* column 1, line 55 - line 65 *<br>* column 2, line 5 - column 3, line 22 *<br>--- | 1-10 | A01N1/02<br>A61K37/02 |
| Y | WO-A-8 700 753 (LINDSTROM R.L. ET AL)<br>* claims 1-2 *<br>--- | 1-10 | |
| Y | WO-A-8 502 975 (LINDSTROM R.L. ET AL)<br>* page 3, line 18 - line 20 *<br>* page 2, line 11 - line 22 *<br>* page 7, line 4 - line 7 *<br>* page 9, line 16 - line 20 *<br>--- | 1-10 | |
| Y | PATENT ABSTRACTS OF JAPAN<br>vol. 8, no. 162 (C-235)(1599) 26 July 1984<br>& JP-A-59 065 020 ( JIEE SHII AARU ) 13 April 1984<br>* abstract *<br>--- | 1-10 | |
| Y | WORLD PATENTS INDEX<br>Week 7103,<br>Derwent Publications Ltd., London, GB;<br>AN 71-05400S<br>& JP-B-46 002 036 (MIZUKAWA, T. ET AL)<br>* abstract *<br>--- | 1-10 | **TECHNICAL FIELDS SEARCHED (Int. Cl.5 )**<br><br>A01N<br>A61K |
| A | EP-A-0 140 998 (J.C.R.KABUSHIKI KAISHA)<br>* the whole document *<br><br>----- | 1-10 | |

The present search report has been drawn up for all claims

| Place of search | Date of completion of the search | Examiner |
|---|---|---|
| BERLIN | 03 FEBRUARY 1992 | AVEDIKIAN P.F. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...........................................................................................
& : member of the same patent family, corresponding
    document